# EUROPEAN PATENT SPECIFICATION

(11) **EP 3 669 746 B1**
(45) Date of publication and mention of the grant of the patent: **12.08.2026**
(21) Application number: 18215324.7
(22) Date of filing: 21.12.2018
(51) Int. Cl.: A61B 1/005, A61M 25/01, A61B 1/00

(54) **AN ARTICULATED TIP PART FOR AN ENDOSCOPE**
GELENKIGER SPITZENTEIL FÜR EIN ENDOSKOP
PARTIE DE POINTE ARTICULÉE POUR UN ENDOSCOPE

(43) Date of publication of application: 24.06.2020
(73) Proprietor: Ambu A/S, 2750 Ballerup (DK)
(72) Inventor: Jensen, Thomas Bachgaard, 1721 Copenhagen V (DK)
(74) Representative: AWA Denmark A/S

(56) References cited:
- EP-A1- 1 563 784
- DE-A1- 4 222 817
- US-A1- 2012 215 068
- US-B1- 6 171 235
- US-B1- 6 547 721

## Description

The present invention relates to an endoscope, and more specifically to an articulated tip part for an endoscope.

Endoscopes are well known for visually inspecting inaccessible places such as human body cavities. Typically, the endoscope comprises an elongated insertion tube with a handle at the proximal end, as seen from the operator, and a tip part normally with a visual inspection device, such as a built-in camera, at the distal end of the elongated insertion tube. This definition of the terms distal and proximal, i.e. proximal being the end closest to the operator along the extent of the endoscope and distal being the end remote from the operator along the extent of the endoscope, as used herein for endoscopes in general, is adhered to in the present specification.

As the name indicates, endoscopes are used for seeing inside things, such as lungs or other human body cavities of a patient. Modern endoscopes are therefore typically equipped with a light source and a vision receptor including a vision sensor, such as a camera or an image sensor. Provided that sufficient light is present, it is possible for the operator to see where the endoscope is steered and to set the target of interest once the tip has been advanced thereto. This therefore normally requires illumination of the area in front of the distal tip of the endoscope, in particular the field of vision of the camera(s). The light source, such as a light emitting diode or an optical fibre, may provide illumination.

Electrical wiring for the camera and other electronics, such as LED lighting accommodated in the tip part at the distal end, run along the inside of the elongated insertion tube from the handle to the tip part. Instead of using cameras, endoscopes may also be fibre-optic, in which case the optical fibres run along the inside of the elongated insertion tube to the tip part. A tubular sleeve may cover the tip part. For some applications, a working or suction channel may run along the inside of the insertion tube from the handle to the tip part, e.g. allowing liquid to be removed from the body cavity or allowing for insertion of surgical instruments or the like, into the body cavity. The suction channel may be connected to a suction connector, typically positioned at a handle at the proximal end of the insertion tube. For other applications, the working or suction channel may be omitted.

In order to be able to manoeuvre the endoscope inside the body cavity, the distal end of the endoscope may comprise a bending section with increased flexibility, e.g. an articulated tip part allowing the operator to bend this section. Typically, this is done by tensioning or slacking steering wires also running along the inside of the elongated insertion tube from the articulated tip part to a control mechanism of the handle. Furthermore, a working channel may run along the inside of the insertion tube from the handle to the tip part, e.g. allowing liquid to be removed from the body cavity or allowing the insertion of surgical instruments or the like into the body cavity.

A general desire in the field of endoscopy is to electrically insulate the insertion tube, and thus the tip part, from the exterior, so as to prevent or at least reduce the risk of an insulation breakdown and a resulting excessive leakage current. In particular it is desired that electrically conductive components, such as metal components, are positioned at a distance to the outer circumference of the insertion tube.

A general desire in the field of endoscopy is provide a tip part which is sealed to be liquid tight, so as to reduce or prevent liquid ingress into the tip part, and specifically onto any electrical components of the tip part.

A general desire in the field of endoscopy is to examine ever smaller body cavities as this may open up new fields of applications, for instance endoscopes for Ear, Nose, and Throat (ENT) endoscopy, such as rhinoscopy or rhinolaryn-goscopy, which requires an insertion tube with a smaller footprint than for instance bronchoscopy.

On this background, it may be seen as an object of the present invention to provide an improved method of assembling an articulated tip part for an endoscope.

One or more of these objects may be met by the present specification as described in the following.

The present specification relates to a bendable articulated tip part for an endoscope, the articulated tip part comprising:
a sub-assembly including a bending section comprising a number of hingedly connected segments including a proximal end segment, a distal end segment, and a plurality of intermediate segments positioned between the proximal end segment and the distal end segment, the sub-assembly further includes a circumferentially extending, outer flange surface which extends from a first outer circumference of the sub-assembly to a second, smaller, outer circumference of the sub-assembly; and
a tubular sleeve including a rim surface, the tubular sleeve at least covering the intermediate segments of the bending section;
wherein the rim surface of the tubular sleeve faces the flange surface of the sub-assembly.

An advantage of a tip part according to this specification may be that by having the tubular sleeve only extending along a portion of the full length of the tip part, allows the provision of a tip part with a smaller cross-sectional area while still keeping the benefits of the tubular sleeve, which may include a smooth outer surface and increased sealing of the tip part. This may be achieved by providing the tubular bending to not cover elements of the tip part having a relatively large cross-sectional area. A tip part with a smaller cross-sectional area may also increase patient wellbeing while undergoing endoscopy since a smaller insertion tube may be even less intrusive for current applications.

Additionally or alternatively, a component of a first direction perpendicular to the rim surface in the proximal-distal direction may be in an opposite direction relative to a component of a second direction perpendicular to the flange surface in the proximal-distal direction.

Additionally or alternatively, a first direction perpendicular to the rim surface in the proximal-distal direction and a second direction perpendicular to the flange surface may intersect between the flange surface and the rim surface.

Additionally or alternatively, the rim surface of the tubular sleeve may be a surface extending in a radial direction potentially of the proximal-distal axis.

Additionally or alternatively, the flange surface may be extending in a substantially tangential direction of the proximal-distal axis or a central axis of the tip part. The flange surface may extend around the entire circumference of the sub-assembly. The flange surface may face an axial direction of the tip part. At least a portion of the flange surface may be substantially axial, for instance by having a normal direction with a component parallel to the proximal-distal axis.

Additionally or alternatively, the flange surface may taper off from a first outer circumference of the sub-assembly to a second, smaller, outer circumference of the sub-assembly.

Additionally or alternatively, the flange surface may taper in circumference or diameter potentially from the first circumference to the second circumference. The flange surface may extend from the first outer circumference to the second, smaller, outer circumference by tapering off gradually or continuously. Alternatively, the flange surface may extend discontinuously or abruptly, potentially to form a 90-degree step. The flange surface may taper with an angle, potentially measured in relation to the central, proximal-distal axis, the angle may be equal to or less than 75, 80, 85, or 90 degrees.

Additionally or alternatively, the second circumference may be adjacent to the first circumference potentially separated by the flange surface.

Additionally or alternatively, the first and second circumference may form the boundaries between which the flange surface extends.

Additionally or alternatively, the difference in the diameter of the first and second circumference may be substantially equal to twice the thickness of the tubular sleeve.

In some embodiments, the tip part may be a single-use or disposable tip part, potentially for a single-use or disposable endoscope, and may not be intended for cleaning and/or reusing.

Additionally or alternatively, an outer circumference of the bendable articulated tip part may be substantially uniform along the length of the bending section.

The bending section may be a section allowing the tip part to bend relative to non-bendable parts of the insertion tube, for instance the flexible tube or cap, potentially so as to allow an operator to manipulate the tip part, potentially by operating a control element of an operating handle, while inserted into a body cavity of a patient.

Additionally or alternatively, the bending section may be integrally formed, potentially in one piece.

At least one hinge member may interconnect adjacent segments with each other, e.g. the proximal end segment with an adjacent intermediate segment and the distal end segment with an adjacent intermediate segment.

Additionally or alternatively, each pair of adjacent segments may be interconnected by at least one, two, or three hinge members. The hinge member(s) may be bridging a gap between adjacent segments. The hinge member may allow adjacent segments to pivot relative to each other to allow the bending section to bend.

Each segment may comprise a proximal surface, potentially except the proximal end segment, facing a distal surface of an adjacent segment forming a gap therein between, and at least one hinge member may bridge the gap. Each segment may comprise a distal surface, potentially except the distal end segment, facing a proximal surface of an adjacent segment forming a gap therein between, and at least one hinge member may bridge the gap. The proximal surface and/or distal surface of each segment may be substantially planar.

Each segment of the bending section may comprise a similar, potentially substantially equal, outer, surface, potentially circumferentially extending around a central, proximal-distal axis of the bending section or tip part. The segments may be substantially cylindrically and/or disc-shaped. The outer surface of each segment may form part of an outer circumferentially extending side wall, which may extend around a central axis, potentially a proximal-distal axis, of the tip part. Each segment may be substantially cylindrical disc-shaped. Each segment may be provided so that the bending section has a uniform outer contour.

Additionally or alternatively, each hingedly interconnected segment may consist essentially of the same material and may be integrally formed, potentially in one piece.

Additionally or alternatively, the hingedly interconnected segments may comprise, or consist essentially of, polypropylene (PP), polyethylene (PE), or polyoxymethylene (POM).

The tubular sleeve may seal the connection between the bending section and an adjacent element of the tip part, for instance a flexible tube and/or a cap. The tubular sleeve may seal any gaps between adjacent segments. The tubular sleeve may provide the tip part and/or the flexible tube with an outer surface configured for insertion into a body cavity, for instance a substantially smooth outer surface.

Additionally or alternatively, an outer surface of the tubular sleeve may be arranged substantially flush with an outer surface of the sub-assembly bounded by the first circumference.

Additionally or alternatively, the sub-assembly may not comprise the tubular sleeve.

Additionally or alternatively, the wall thickness of the tubular sleeve may be less than 0.3, 0.25, 0.2, 0.15, 0.1, 0.9, or 0.8 mm.

The tubular sleeve may be made of a translucent, potentially transparent, material.

Additionally or alternatively, the tubular sleeve may cover a gap, potentially all gaps, between adjacent segments of the bending section.

Additionally or alternatively, the distal rim surface of the tubular sleeve may be positioned proximally in relation to the distal rim surface of bendable articulated tip part.

Additionally or alternatively, the proximal rim surface of the tubular sleeve may be positioned distally in relation to a proximal end of the proximal end segment.

Additionally or alternatively, a maximum diameter of the first circumference may be greater than a maximum diameter of the second circumference. The maximum diameter of inner circumference of the tubular sleeve may be substantially equal to the maximum diameter of the second circumference.

Additionally or alternatively, the maximum diameter of the sleeve surface may substantially equal to the maximum diameter of at least one, potentially all, of the intermediate segments.

The sub-assembly includes a sleeve surface which comprises a circumferentially extending outer surface of each of the intermediate segments and is bounded at one end by the second circumference. The sleeve surface may have a substantially constant circumference along a proximal-distal axis of the tip part. The tubular sleeve covers at least a portion of the sleeve surface.

The second circumference may form a boundary of the sleeve surface. The sleeve surface may have a substantially constant circumference, potentially excluding any gaps between gaps of adjacent segments in which the sleeve surface may be discontinuous. The maximum diameter of the sleeve surface may be substantially constant, potentially excluding any gap between segments of the bending section. The substantially constant circumference of the sleeve surface may be narrower in relation to an outer circumference of remaining parts of the sub-assembly, for instance the cap and/or flexible tube. Circumference or diameter variations of less than plus/minus 5% may still be considered substantially constant.

The tubular sleeve may not overlap with the image sensor of the camera assembly and/or the distal end of the flexible tube.

Additionally or alternative, the sleeve surface may form a floor of a recess or cut-out of the sub-assembly. At least one, potentially both, of the rim surfaces of the tubular sleeve may be positioned in the recess or cut-out.

Additionally or alternatively, the tubular sleeve may seal a gap, potentially all gaps, between adjacent segments of the bending section. The proximal end of the proximal segment, may be sealed by another component of the tip part, for instance a cap. The distal end of the distal segment may be sealed by another component of the tip part, for instance a flexible tube.

The sub-assembly may include a proximal, circumferentially extending, outer flange surface which may taper from a first outer circumference of the sub-assembly to a second, smaller, outer circumference of the sub-assembly, the proximal flange surface may be positioned at or adjacent to the proximal end segment.

Additionally or alternatively, the first outer circumference of the sub-assembly may be positioned proximally in relation to the second, smaller, outer circumference of the sub-assembly.

Additionally or alternatively, the proximal flange surface and the sleeve surface may be positioned adjacently. The proximal flange surface and the sleeve surface may be directly connected.

Additionally or alternatively, the proximal rim surface of the tubular sleeve may be positioned adjacent to the proximal flange surface.

Additionally or alternatively, the proximal flange surface may extend around the entire circumference of the tip part.

Additionally or alternatively, the outer surface of the tubular sleeve may be arranged substantially flush with the first circumference or an outer surface of the sub-assembly adjacent to the first circumference.

Additionally or alternatively, the proximal flange surface may taper off from a first outer circumference of the sub-assembly to a second, smaller, outer circumference of the sub-assembly.

Additionally or alternatively, the proximal flange surface may taper off in circumference or diameter potentially from the first circumference to the second circumference. The proximal flange surface may extend from the first outer circumference to the second, smaller, outer circumference by tapering off gradually or continuously. The proximal flange surface may extend discontinuously or abruptly, potentially to form a 90-degree step. The proximal flange surface may taper with an angle, potentially measured in relation to the central, proximal-distal axis, the angle may be equal to or less than 75, 80, 85, or 90 degrees. The proximal flange surface may form a step potentially from the third circumference to the fourth circumference.

The proximal flange surface may be forming part of, potentially forming an integral part of or integrally formed in one piece by, an outer, circumferentially extending surface of the proximal end segment.

In some embodiments, the tip part may further comprise a flexible tube having a distal end connected to the proximal end of the proximal end segment, wherein the proximal rim surface of the tubular sleeve and/or the proximal flange surface may be positioned distally in relation to the distal end of the flexible tube.

In these embodiments, the distal end of the flexible tube may at least partially define the lower bound for minimizing the maximum circumference or diameter of the tip part, thus by positioning the proximal rim surface of the tubular sleeve in this way, a smaller tip part may be provided.

Additionally or alternatively, the proximal rim surface of the tubular sleeve may be positioned distally in relation to and/or positioned at a distance from the distal end of the flexible tube potentially so that an outer circumference of the bendable articulated tip part along the length of the bending section is substantially uniform.

Additionally, the flexible tube may be positioned in a spacing of the proximal end segment.

Additionally, the flexible tube may be attached or secured to the proximal end segment potentially by an adhesive.

The flexible tube may comprise a circumferentially extending, side wall including an inner surface and an outer surface. The inner surface may define an interior spacing. The flexible tube may comprise a distal end, which may be connected to the proximal end segment of the bending section. The flexible tube may comprise a proximal end configured for connection with remaining parts of the endoscope, for instance an operating handle of the endoscope. The flexible tube may be integrally provided in one piece. The flexible tube may be made of a polymeric material. The flexible tube may surround or enclose the cable passage and/or the working passage and/or the steering wire(s).

The tip part may further comprise a flexible tube having a distal end connected to the proximal end of the proximal end segment, wherein the proximal flange surface may be formed by an overlap of the proximal end segment and the flexible tube.

The tip part may further comprise a flexible tube having a distal end connected to the proximal end of the proximal end segment, wherein the proximal flange surface may be formed at the interconnection of the proximal end segment and the flexible tube.

Additionally or alternatively, the flange surface may not extend to overlap with the distal end of the flexible tube.

The sub-assembly may include a distal circumferentially extending, outer flange surface which extends from a third outer circumference of the sub-assembly to a fourth, smaller, outer circumference of the sub-assembly, the distal flange surface being positioned at or adjacent to the distal end segment.

Additionally or alternatively, the distal flange surface and the sleeve surface may be positioned adjacently. The sleeve surface may be positioned between the proximal flange surface and the distal flange surface. The distal flange surface and the sleeve surface may be directly connected.

Additionally or alternatively, the distal rim surface of the tubular sleeve may be positioned adjacent to the distal flange surface.

Additionally or alternatively, the distal rim surface of the tubular sleeve may face the distal flange surface.

Additionally or alternatively, the proximal and distal rim surfaces are positioned between the distal and proximal flange surfaces.

Additionally or alternatively, the distal flange surface may extend around the entire circumference of the tip part.

Additionally or alternatively, the outer surface of the tubular sleeve may be arranged substantially flush with the third circumference or an adjacent surface of the third circumference.

Additionally or alternatively, the distal flange surface may taper off from a third outer circumference of the sub-assembly to a fourth, smaller, outer circumference of the sub-assembly.

Additionally or alternatively, the distal flange surface may taper off in circumference or diameter potentially from the third circumference to the fourth circumference. The distal flange surface may extend from the first outer circumference to the second, smaller, outer circumference by tapering off gradually or continuously. The distal flange surface may extend discontinuously or abruptly, potentially to form a 90-degree step. The distal flange surface may taper with an angle, potentially measured in relation to the central, proximal-distal axis, the angle may be equal to or less than 75, 80, 85, or 90 degrees. The distal flange surface may form a step potentially from the third circumference to the fourth circumference.

Additionally or alternatively, the third circumference may be substantially equal to the first circumference.

Additionally or alternatively, the fourth circumference may be substantially equal to the second circumference.

Additionally or alternatively, the sleeve surface may be delimited by the second circumference and the fourth circumference and may have a substantially constant circumference between the second and fourth circumference, potentially excluding any gaps between segments.

The distal flange surface may be forming part of, potentially forming an integral part of or formed in one piece with, an outer, circumferentially extending surface of the distal end segment.

The tip part may further comprise a camera assembly, wherein the distal rim surface of the tubular sleeve and/or the distal flange surface may be positioned proximally in relation to an image sensor of the camera assembly and/or a light source of the camera assembly.

The image sensor and/or the light source of the camera assembly may at least partially define the lower bound for minimizing the maximum circumference or diameter of the tip part, thus by positioning the distal rim surface of the tubular sleeve in this way, a smaller tip part may be provided.

Additionally or alternatively, the flange surface may not extend to overlap with the image sensor of the camera assembly. Alternatively, the light source may be a surface configured for emitting light.

The camera assembly may be positioned at a distal end of the tip part and allowing an operator to inspect a body cavity when the tip part is inserted into the body cavity. The camera assembly may be positioned in a spacing of the cap. The camera assembly may comprise one or some or all elements selected from the group consisting of: an image sensor configured to capture an image, at least one lens configured to alter light received by the image sensor, a camera housing for supporting the parts of the camera assembly, at least one light source configured to provide illumination for the image sensor, a printed circuit board, at least one signal cable for carrying an image signal from the camera assembly to the operator, and a power cable for supplying the camera assembly with electricity. The printed circuit board may be configured to process a signal from the image sensor. The signal cable and/or the power cable may be connected to printed circuit board. The signal cable may be configured for transmitting an image signal to the operating handle or an output for a monitor. The power cable may be configured to supply power to the printed circuit board.

The tip part or camera assembly may comprise at least one light source positioned at a distal end of the tip part so that light emitted from the light source is directed distally. At least one or all of the light source(s) may be light emitting diode(s) and/or light fibre(s). The light source(s) may be configured for providing illumination for the image sensor of the camera assembly. The number of light sources may be at least two or at the most two or exactly two.

The tip part may further comprise a cap having a proximal end connected to the distal end of the distal end segment,
wherein the distal rim surface of the tubular sleeve and/or the distal flange surface may be positioned proximally in relation to the proximal end of the cap.

This may provide the advantage that a cap may ensure the liquid tightness and electrical insulation of the distal end of the tip part which may relieve the tubular sleeve of these functions. As the tip part may be made smaller as a cap can be made with smaller dimensions and tight tolerances. Especially when manufactured by injection moulding. This may allow the tubular sleeve to be made shorter and thus may allow the provision of a tip part with a smaller footprint.

Additionally or alternatively, the cap may include a circumferentially extending side wall enclosing a spacing potentially accommodating the camera assembly. The camera assembly may be positioned to view out of a distal end of the cap. The distal end of cap may form the distal end of tip part. The cap may be attached or secured to the distal end segment potentially by an adhesive.

Additionally or alternatively, the cap may have a proximal end positioned adjacent to the distal end segment. The cap may have a distal end, potentially forming the distal end of the tip part. The cap may comprise an outer circumferentially extending side wall enclosing a spacing. The cap may accommodate a camera assembly, potentially positioned in the spacing of the cap. The cap may comprise an end wall positioned at the distal end of the cap. The end wall may comprise a window, which may allow light to propagate there through to an image sensor of the camera assembly. The end wall may comprise an opening, potentially adjacent to the camera assembly, so that light passing through the opening is received by an image sensor of the camera assembly.

Additionally or alternatively, the outer surface of the tubular sleeve may be arranged substantially flush with the outer surface of the outer circumferentially extending side wall of the cap.

The tip part may further comprise a cap having a proximal end connected to the distal end of the distal end segment, wherein the distal flange surface is formed by an overlap of the distal end segment and the cap or is formed by the interconnection of the distal end of the distal end segment and the proximal end of the cap.

Additionally or alternatively, the distal flange surface may be formed by a proximal rim surface of the cap.

The tubular sleeve may not overlap or cover a portion of the cap and/or a portion of the flexible tube.

In the present invention, , the tubular sleeve may only cover or overlap with at least a portion of the sleeve surface.

Additionally or alternatively, the tubular sleeve may not cover or overlap with the cap and/or flexible tube.

The tubular sleeve may be secured to the bending section, potentially by an adhesive.

The flexible tube, the cap, and the bending section may be provided separately, and/or as separate components that are attached to each other. The flexible tube and the bending section may be formed as separate parts. The cap and the bending section may be formed as separate parts.

The bending section may comprise, potentially consist essentially of, a first material, and the flexible tube may comprise, potentially consist essentially of, a second, different material, and the cap may comprise, potentially consist essentially of, a third, different material. The flexible tube may be made of a polyolefin, potentially a plastic polymer. The cap may be made of a polyolefin, potentially a plastic polymer. The flexible tube and the cap may be made of different materials.

The bendable articulated tip part and/or the bending section and/or the cap and/or the flexible tube may form part of an insertion tube. The insertion tube or a distal end thereof may be suitable for insertion into a body cavity, potentially a lung, through a body opening, potentially a mouth. The body may be a natural and/or artificial body, potentially a human or animal body. The insertion tube may be connected to and extend from an operating handle towards a distal end of the endoscope. The tip part may be positioned at and form the distal end of the insertion tube.

The tip part and/or the segments may comprise at least one cable passage for accommodating at least one cable, e.g. a signal cable for carrying an image signal and/or a power cable for carrying electricity. The cable passage may comprise a through hole in each of the segments, potentially so as to form a cable passage, that may be extending from the distal end segment through the intermediate segment(s) to the proximal end segment. The cable passage may be positioned adjacent to a centre of the segments. The tip part may comprise a signal cable for carrying an image signal and/or a power cable for carrying electricity, the signal and/or the power cable may be positioned in the cable passage.

The tip part may comprise a working passage. The working passage may be configured for accommodating a tube providing a working channel. The working passage may be different from the cable passage. The working channel may be a suction channel for providing a suction at the distal end of the tip part. The suction channel may be connected to a suction connector, potentially at a handle at the proximal end of the insertion tube. The working channel may allow insertion of surgical instruments there through to the distal end of the tip part. The working passage may be omitted to minimize the footprint of the tip part.

The tip part may comprise at least one steering wire. The steering wire may further be positioned in a steering wire passage of the tip part. The steering wire passage may be formed by a number of through holes provided in the segments of the tip part. The steering wire passage may be different from the cable passage and/or the working passage. An end of the steering wire may be secured in the distal end of the tip part, and another end of the steering wire may be connected to a control element, potentially connected to a control lever of the control element. Thus by manipulating the control element the steering wire may be tensioned on one side of the plane of the hinge members, and slacked on the other, thus allowing the bending section to bend in a desired direction.

The steering wire may be a first steering wire and the articulated tip part may further comprise a second steering wire, potentially provided similarly to the first steering wire. The second steering wire may be positioned in a second steering wire passage.

The tip part may form part of an endoscope.

The term "endoscope" may be defined as a device suitable for examination of natural and/or artificial body openings, e.g. for exploration of a lung cavity. Additionally, or alternatively, the term "endoscope" may be defined as a medical device.

The endoscope may be a single-use or disposable endoscope and may not be intended for being cleaned and/or reused.

The endoscope may comprise a control element. The control element may be configured to allow an operator to control the steerable tip part of the insertion tube by the at least one steering wire. The control element may allow bending the articulated tip part in at least one direction, potentially in two directions, the two directions potentially being opposite. The control element may be accommodated in the operating handle. The control element may include a lever allowing an operator to control the control element. The lever may extend outwardly from the control element, potentially through the operating handle. The control element may be in the form of a roller or a roller disc.

The endoscope may comprise an operating handle. The operating handle may be suitable for allowing an operator to grip and to operate the endoscope, potentially with one hand. The operating handle may comprise a handle housing arranged at a proximal end of the insertion tube. The handle housing may accommodate the control element.

The present specification also relates to a system for visually inspecting inaccessible places such as human body cavities, the system comprising:
an endoscope with a tip part according to the first aspect, and a monitor, wherein the endoscope is connectable to the monitor, and the monitor allow an operator to view an image captured by the camera assembly of the endoscope.

The present invention relates to a method comprising the steps of:
providing a bending section and comprising a number of hingedly connected segments including a proximal end segment, a distal end segment, and a plurality of intermediate segments positioned between the proximal end segment and the distal end segment, the sub-assembly further includes a circumferentially extending, outer flange surface which extends from a first outer circumference of the sub-assembly to a second, smaller, outer circumference of the sub-assembly;
providing a tubular sleeve comprising a distal rim surface and/or a proximal rim surface and an inner circumference delimiting an interior spacing;
applying a pressure differential with a greater pressure on an interior side of the tubular sleeve relative to an exterior side of the tubular sleeve, so as to increase an inner circumference of the tubular sleeve;
inserting the bending section into the interior spacing of the tubular sleeve; and
relieve the pressure differential to decrease the inner circumference of the tubular bending, so that the tubular sleeve covers the intermediate segments and the rim surface of the tubular sleeve faces the flange surface of the sub-assembly.

The step of providing a bending section and the step of providing a tubular sleeve may be a step of providing a tip part according to the present specification.

Additionally, the method may comprise a step of sealing a gap between the bending section and the distal end and/or proximal end of the tubular sleeve, potentially by applying an adhesive and further potentially causing or allowing the adhesive to harden.

Additionally, the method may comprise a step of securing the distal end and/or proximal end of the tubular sleeve to the bending section, potentially by applying an adhesive and further potentially causing or allowing the adhesive to harden.

In this specification, the term "outer surface" or "exterior surface" may be understood as a surface configured for facing a body cavity when the tip part is inserted into a body and outwardly relative to the central, proximal-distal axis of the tip part.

In this specification, the term "cross-section" or "cross-sectional" may be understood to define a cross-section being normal or perpendicular to a central, proximal-distal axis of the tip part or to a length direction of the tip part.

In this specification, the term "diameter" may be defined as a straight line in a plane normal to a centre axis of a body, the line starting from one side of the body intersecting the centre axis of the body and terminates at another side of the body. For example, the body may be the bending section. In this case, the diameter of the bending section may be defined as the length of a straight line perpendicular to the central, proximal-distal axis of the bending section extending from one side of the bending section, intersecting through the central, proximal-distal axis, and terminating at another opposite side of the bending section. A maximum diameter may be defined as the diameter with the maximum length of any diameter in the associated plane of the associated body, for instance the maximum diameter of an ellipse may in this specification understood to be the major diameter.

In this specification, the proximal-distal axis may be defined as a central axis of the tip part from the proximal end to the distal end, for instance when the tip part is substantially cylindrically shaped, the proximal-distal axis may follow the centre line of this cylindrical shape. The proximal-distal axis may follow the shape of the tip part, so that if the bending section of the tip part is bent, then the proximal-distal axis follows the bending shape of the tip part.

A person skilled in the art will appreciate that any one or more of the above aspects of the invention and embodiments thereof may be combined with any one or more of the other aspects of the invention and embodiments thereof. For instance, the sub-assembly may include another flange surface.

The invention will now be described in greater detail based on non-limiting exemplary embodiments and with reference to the drawings, on which:
Fig. 1a shows a perspective view of an endoscope in which a tip part according to the present disclosure is implemented,
Fig. 1b shows a perspective view of a monitor to which the endoscope of Fig. 1a is connectable,
Fig. 2 shows a perspective view of a tip part,
Fig. 3 shows a side view of a sub-assembly of the tip part of Fig. 2,
Fig. 4a shows a side detail view of the proximal flange surface of the detail view A of Fig. 3,
Fig. 4b shows a cross-sectional view of the proximal flange surface along cross-section plane C-C of Fig. 4a in the proximal direction,
Fig. 4c shows a side detail view of the distal flange surface of the detail view B of Fig. 3,
Fig. 4d shows a cross-sectional view of the distal flange surface along cross-section plane D-D of Fig. 4c in the distal direction,
Fig. 5a shows a side view of the tip part of Fig. 2,
Fig. 5b shows a cross-sectional view of the proximal flange surface along cross-section plane E-E of Fig. 5a in a radial direction,
Fig. 5c shows a cross-sectional view of the distal flange surface along cross-section plane F-F of Fig. 5a in a radial direction,

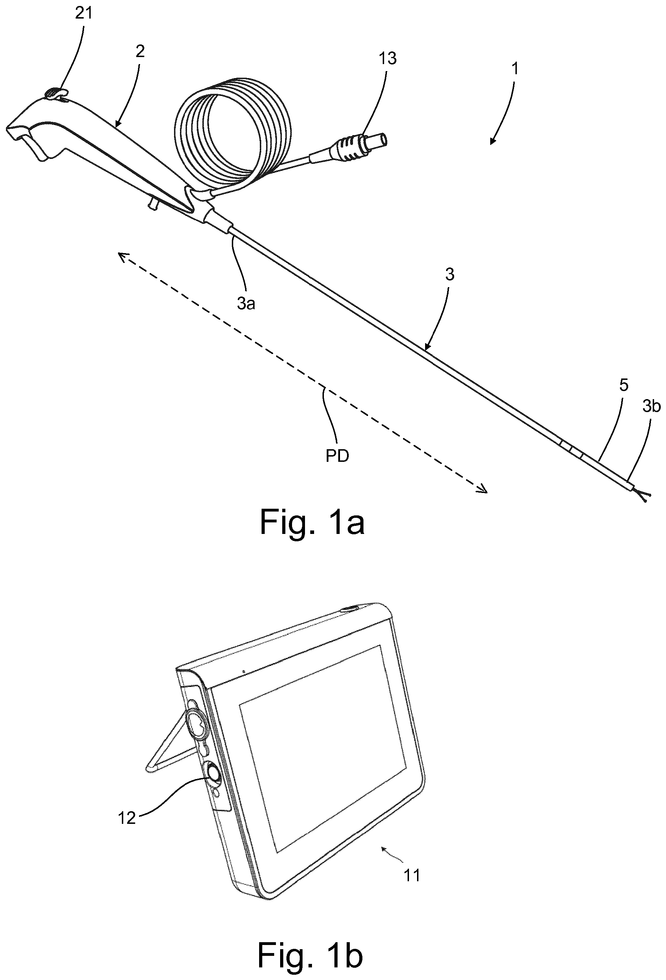

Referring first to Fig. 1a, an endoscope 1 is shown. The endoscope is disposable, and not intended to be cleaned and reused. The endoscope 1 comprises an elongated insertion tube 3. The insertion tube 3 is suitable for insertion into a lung of a body through a mouth. The body can be a natural or artificial body, for instance a human or animal body. At the proximal end 3a of the insertion tube 3 an operating handle 2 is arranged. The operating handle 2 has a control lever 21 for manoeuvring an articulated tip part 5 at the distal end 3b of the insertion tube 3 by means of a steering wire 31a. A camera assembly 61 is positioned in the tip part 5 and is configured to transmit an image signal through a monitor cable 13 of the endoscope 1 to a monitor 13.

In Fig. 1b, a monitor 11 is shown. The monitor 11 may allow an operator to view an image captured by the camera assembly of the endoscope 1. The monitor 11 comprises a cable socket 12 to which a monitor cable 13 of the endoscope 1 can be connected to establish a signal communication between the camera assembly 61 of the endoscope 1 and the monitor 11.

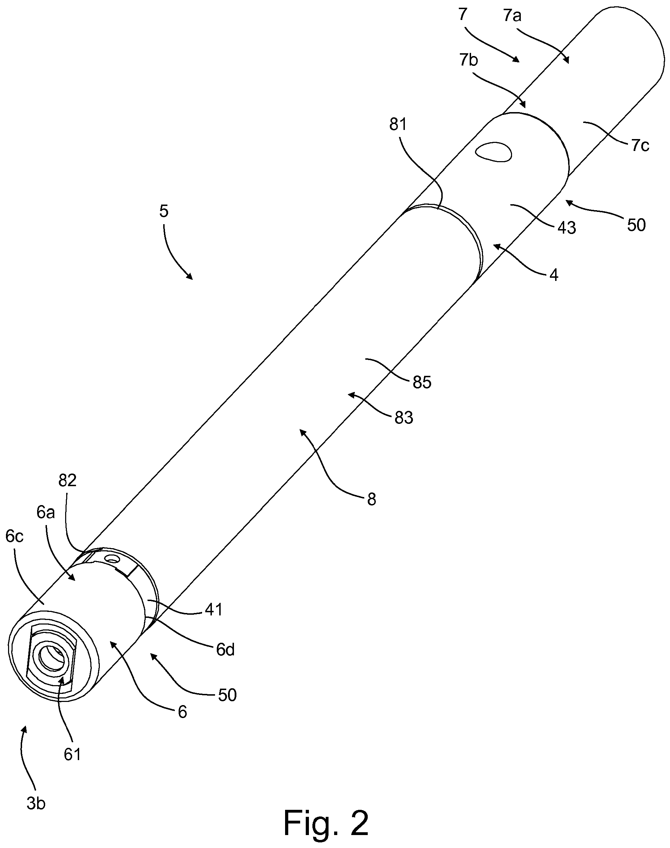

Turning to Fig. 2, the distal end 3b of the insertion tube 3 is shown and more specifically the articulated tip part 5. The tip part comprises a sub-assembly 50 including a bending section 4, a cap 6, a camera assembly 6, and a flexible tube 7. A tubular sleeve 8 wraps around the bending section 4 of the sub-assembly 50. The tip part 5 has a substantially uniform outer circumference from the distal end 3b of the tip part 5 to the proximal end of the proximal end segment. The bending section 4 allows the tip part 5 to bend relative to the flexible tube 7, so as to allow an operator to manipulate the tip part 5 while inserted into a body cavity.

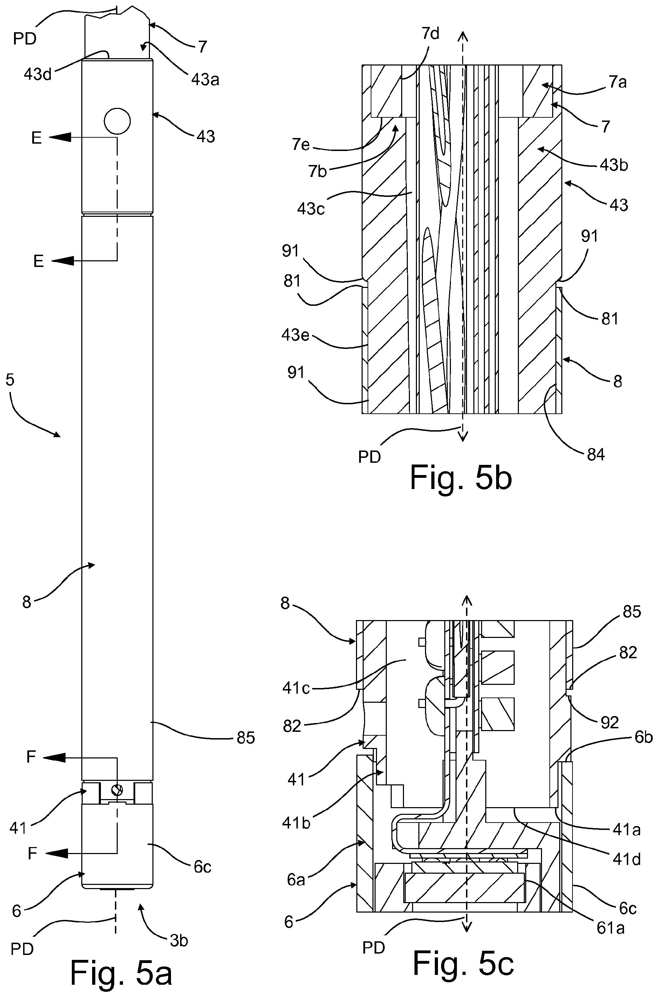

As seen in Figs. 2, 4c, and 5c, the cap 6 includes a circumferentially extending side wall 6a enclosing a spacing accommodating the camera assembly 61. The distal end of cap forms the distal end 3b of insertion tube 3 and the tip part 5. The proximal end 6b of the cap 6 is positioned adjacent and is secured to the distal end segment 41 by a hardened adhesive positioned in a gap in the overlap of the proximal end 6b of the cap 6 and the distal end 41a of the distal end segment 41 as better seen in Fig 5c. The outer surface 85 of the tubular sleeve 8 is arranged substantially flush with the outer surface 6c of the outer circumferentially extending side wall 6a of the cap 6.

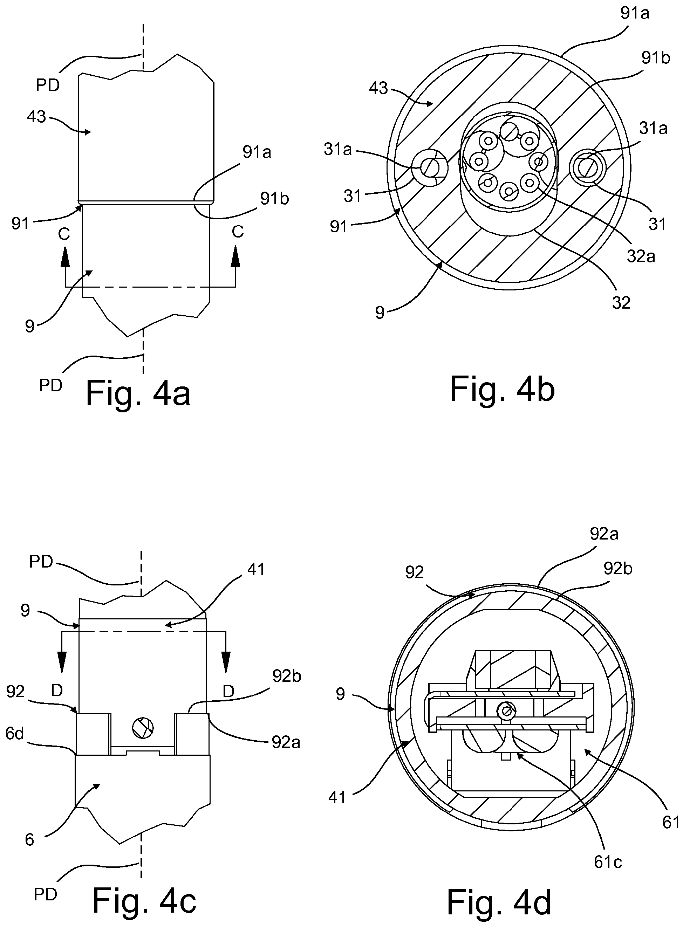

As seen in Figs. 1-3, the camera assembly 61 is positioned at a distal end 3b of the insertion tube 3 and tip part 5 and allows an operator to inspect a body cavity when the insertion tube 3 is inserted into the body cavity. As best seen in Figs. 4d and 5c, the camera assembly 61 comprises an image sensor 61a configured to capture an image, at least one lens (not shown) configured to alter light received by the image sensor 61a, a camera housing (not shown) for supporting the parts of the camera assembly 61, two light sources (not shown) configured to provide illumination for the image sensor 61a in the form of light emitting diodes, a printed circuit board 61c, signal cables configured for carrying an image signal from the camera assembly 61 to the operator, and power cables configured for supplying power to the printed circuit board 61c. As can be seen in the centre of Fig. 4b and 5c, the signal cables and the power cables are connected to printed circuit board 61c. The printed circuit board 61c is configured to process a signal from the image sensor 61a and transmit the signal via signal cables to the cable socket 12 for output to a monitor 11. The camera assembly 61 is positioned in the spacing of the cap 6 and is positioned in an opening in a distal end wall of the cap 6 so that the image sensor 61a can receive an image from a distal position outside the cap.

As can be seen in Figs. 2 and 5b, the flexible tube 7 comprises circumferentially extending, side wall 7a with an outer surface 7c and an inner surface 7d. The inner surface 7d defines and encloses an interior spacing. The flexible tube comprises a distal rim surface 7e at a distal end 7b. The flexible tube 7 comprises a proximal end configured for connection with the operating handle 2 of the endoscope 1. The flexible tube 7 is integrally provided in one piece and consists essentially of a second, polymeric material different from the bending section 4. The flexible tube 7 surrounds and encloses the cable passage 32 and the steering wires 31a. The flexible tube 7 is positioned by insertion into the spacing 43c of the proximal end segment 43 through the opening 43d so that the distal end of the flexible tube 7 and the proximal end 43a of the proximal end segment 43 overlaps as best seen in Fig. 5b. The distal end of the flexible tube 7 is secured to the proximal end 43a of the proximal end segment 43 by a hardened adhesive.

As best seen in Figs. 2, 5a, 5b, and 5c, the tip part 5 further comprises a tubular sleeve 8 including a circumferentially extending, side wall 83 with an outer surface 85 and an inner surface 84. The circumferentially extending, side wall 83 of the tubular sleeve 8 extends between a distal rim surface 82 and a proximal rim surface 81. The rim surfaces 81, 82 defines the elongated extent of the tubular sleeve 8. The tubular sleeve 8 wraps around and covers a sleeve surface 90 of the sub-assembly 5 extending from the proximal segment 41 to the distal segment 43 via the intermediate segments 42 and thus covers all gaps between adjacent segments of the bending section. The tubular sleeve is attached to the proximal end segment 43 and the distal end segment 41 by a hardened adhesive sealing and adhering the inner surface 84 of the tubular sleeve 8 to the outer surface 43e, 41e of the proximal end segment 43 and the distal end segment 41, respectively, and thereby seals the connections between the bending section 4 and the flexible tube 7 and the cap 6. The outer surface 85 of the tubular sleeve 8 is arranged substantially flush with the remaining outer surface of the tip part 5. The tubular sleeve 8 is made of a transparent polymeric material.

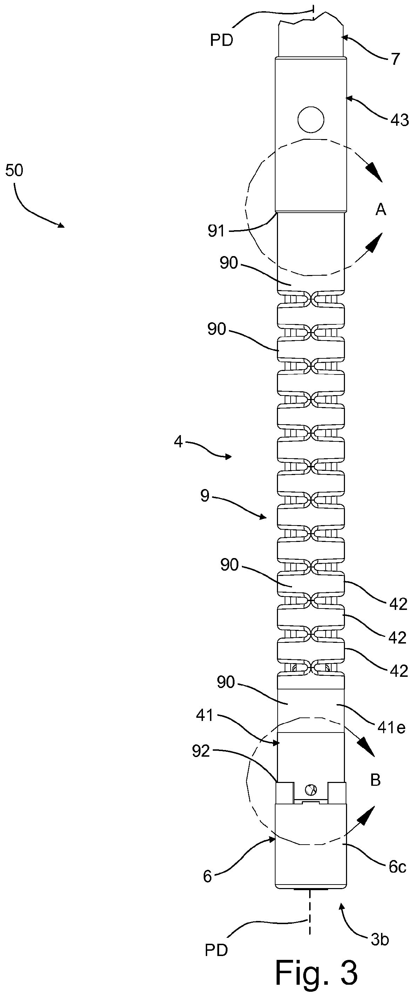

Turning to Fig. 3, the bending section 4 comprises a number of hingedly connected segments including a proximal end segment 43, a distal end segment 41, and a plurality of intermediate segments 42 positioned between the proximal end segment 43 and the distal end segment 41. The number of intermediate segments 42 may be eleven, but may in principle be less or even greater. The proximal end segment 43 comprises an outer, circumferentially extending, side wall 43b enclosing a spacing 43c into which an opening 43d in the proximal end 43a provides access. The side wall 43b includes an outer surface 43e. The proximal end segment 43 is attached to the distal end 7b of the flexible tube 7. The distal end segment 41 comprises an outer, circumferentially extending, side wall 41b enclosing a spacing 41c into which an opening 41d in the distal end 41a provides access. The side wall 41b includes an outer surface 41e. Two hinge members of living hinge type interconnects adjacent segments with each other. The hinge members bridge a gap between adjacent segments. Each segment 41, 42, 43 is cylindrically disc-shaped having an outer, circumferentially extending recess or cut-out 9 with a sleeve surface 90, so that sleeve surface 90 of the bending section 4 forms a substantially uniform outer diameter of the bending section 4. The proximal end 43a of the proximal end segment 43 and the distal end 41a of the distal end segment 41 have a slightly larger outer diameter. The bending section 4 and each hingedly interconnected segment 41, 42, 43 consist essentially of the same material and are integrally formed in one piece. The material is polypropylene (PP) but may be any suitable material, such as polyethylene (PE) or polyoxymethylene (POM).

Furthermore, the sub-assembly 50 includes a proximal, outer flange surface 91 extending around the entire circumference of the proximal end segment 43. The proximal flange surface 91 is positioned at and is integrally formed in one piece by the outer, circumferentially extending surface 43e of the proximal end segment 43.

Furthermore, the sub-assembly 50 also includes a distal, outer flange surface 92 extending around the entire circumference of the distal end segment 41. The distal flange surface 92 is positioned at and is integrally formed in one piece by the outer, circumferentially extending surface 41e of the proximal end segment 41.

As best seen in Fig. 3, the proximal flange surface 91 and the distal flange surface 92 delimits the recess or cut-out 9. The floor of the recess or cut-out 9 is formed by the sleeve surface 90 extending circumferentially around the bending section 4. The circumferentially extending, outer surface of each of the intermediate segments forms part of the sleeve surface 90 along with narrow portions adjacent of the end segments 41, 43 adjacent to the flange surfaces 91, 92. The sleeve surface 90 thus has a substantially constant circumference along the proximal-distal axis of the tip part excluding the gaps between adjacent segments 41, 42, 43. The gaps between adjacent segments 41, 42, 43 forms breaks in the sleeve surface 90. An outer circumference of the sleeve surface 90 is substantially narrower than the outer circumference of the remaining parts of the bending section 4 and cap 6 to leave space for the tubular sleeve 8.

Referring now to Figs. 4a-4b, the proximal flange surface 91 extends from a first, proximal, outer circumference 91a of the proximal end segment 43 to a second, smaller, distal, outer circumference 91b of the proximal end segment 43 by gradually tapering off. The flange surface 91 could also extend with other shapes between the circumferences, for instance abruptly or taper linearly off by an angle measured in relation to the central, proximal-distal axis PD or form a step similar to the distal flange surface 92. The proximal flange surface 91 is connected to and directly transitions to the sleeve surface 90 at the second outer circumference 91b.

As best seen in Figs. 4c-4d, the distal flange surface 92 extends abruptly forming a step from a third, proximal, outer circumference 92a of the distal end segment 41 to a fourth, smaller, distal, outer circumference 92b of the distal end segment 41. The flange surface 92 could also extends with other shapes, for instance abruptly or taper linearly off by an angle measured in relation to the central, proximal-distal axis PD or continuously. The distal flange surface 92 is connected to and directly transitions to the sleeve surface 90 at the fourth outer circumference 92b. The third circumference 92a could alternatively be provided a proximal rim surface 6d of the cap 6 as the proximal end 6b of the cap 6 could extend further and thus would achieve similar results. As seen in Fig 4d, the distal flange surface 92 does not extend around the entire circumference of the distal end segment 41.

Turning to Figs. 5a-5c, the tubular sleeve 8 is shown positioned in the recess or cut-out 9 formed by the flange surfaces 91, 92 and the sleeve surface 90 and positioned to cover most of the sleeve surface 90. As best seen in Fig. 5b, the proximal rim surface 81 of the sleeve 8 is positioned adjacent and distally in relation to the proximal flange surface 91. Additionally, the proximal rim surface 81 faces and is oriented towards the proximal flange surface 91. Further, the proximal rim surface 81 and the proximal flange surface 91 could be positioned to abut but is shown positioned at a distance from each other. The second circumference 91b of the proximal flange surface 91 is substantially equal to the circumference of the inner surface 84 of the sleeve 8. The first circumference 91a of the proximal flange surface 91 is substantially equal to the circumference of the outer surface 85 of the sleeve 8 to ensure a substantially uniform outer circumference along the length of the tip part 5 including the bending section 4, however in practice there might be slight differences. A difference of less than plus/minus 5% may be considered substantially equal.

The lower bound of the maximum outer circumference or diameter is partially defined by the distal end 7b of the flexible tube 7, thus by positioning the proximal rim surface 81 distally in relation the distal end 7b of the flexible tube 7 it is ensured that the sleeve 8 does not overlap with the distal end 7b of the flexible tube 7. In other embodiments, other parts of the sub-assembly 50 may define the lower bound of the maximum outer circumference or diameter of the tip part 5.

As best seen in Fig. 5c, the distal rim surface 82 of the sleeve 8 is positioned adjacent and proximally in relation to the distal flange surface 92. Additionally, the distal rim surface 82 faces and is oriented towards the distal flange surface 92. Further, the distal rim surface 82 and the distal flange surface 92 could be positioned to abut but is shown positioned at a distance from each other. The fourth circumference 92b of the distal flange surface 92 is substantially equal to the circumference of the inner surface 84 of the sleeve 8. The third circumference 92a of the distal flange surface 92 is substantially equal to the circumference of the outer surface 85 of the sleeve 8 to ensure a substantially uniform outer circumference along the length of the tip part 5 including the bending section 4, however in practice there might be slight differences. A difference of less than plus/minus 5% may be considered substantially equal.

The lower bound of the maximum outer circumference or diameter is partially defined by the image sensor 61a of the camera assembly 61, thus by positioning the distal rim surface 82 proximally in relation to the image sensor 61c of the camera assembly 61 and the sleeve 8 does not overlap with the image sensor 61c. In other embodiments, other parts of the sub-assembly 50 may define the lower bound of the maximum outer circumference or diameter of the tip part 5.

The following is a list of reference numerals used throughout this specification. In case of any doubt, the reference numerals of the following list apply.
- 1: endoscope
- 11: monitor
- 12: cable socket
- 13: monitor cable
- 2: handle
- 21: control lever
- 3: insertion tube
- 3a: proximal end
- 3b: distal end
- 31: steering wire passage
- 31a: steering wire
- 32: cable passage
- 32a: cable
- 4: bending section
- 41: distal end segment
- 41a: distal end
- 41b: outer circumferentially extending side wall
- 41c: spacing
- 41d: opening
- 41e: outer surface
- 42: intermediate segment
- 43: proximal end segment
- 43a: proximal end
- 43b: outer circumferentially extending side wall
- 43c: spacing
- 43d: opening
- 43e: outer surface
- 5: tip part
- 50: sub-assembly
- 6: cap
- 6a: circumferentially extending side wall
- 6b: proximal end
- 6c: outer surface
- 6d: proximal rim surface
- 61: camera assembly
- 61a: image sensor
- 61b: light source
- 61c: printed circuit board
- 7: flexible tube
- 7a: circumferentially extending side wall
- 7b: distal end
- 7c: outer surface
- 7d: inner surface
- 7e: distal rim surface
- 8: tubular sleeve
- 81: proximal rim surface
- 82: distal rim surface
- 83: circumferentially extending side wall
- 84: inner surface
- 85: outer surface
- 9: recess or cut-out
- 90: sleeve surface
- 91: proximal flange surface
- 91a: first circumference
- 91b: second circumference
- 92: distal flange surface
- 92a: third circumference
- 92b: fourth circumference
- PD: proximal-distal axis

## Claims

1. A method for assembling a bendable articulated tip part (5) for an endoscope (1), the method comprising the steps of:
providing a sub-assembly (50) including a bending section (4) comprising a number of hingedly connected segments (41, 42, 43) including a proximal end segment (43), a distal end segment (41), and a plurality of intermediate segments (42) positioned between the proximal end segment (43) and the distal end segment (41), the sub-assembly (50) further including a circumferentially extending, outer flange surface (91) which extends from a first outer circumference (91a) of the sub-assembly (50) to a second, smaller, outer circumference (91b) of the sub-assembly (50), where the sub-assembly (50) includes a sleeve surface (90) which comprises a circumferentially extending outer surface of each of the intermediate segments (42) and is bounded at one end by the second circumference (91b);
providing a tubular sleeve (8) comprising a distal rim surface (82) and/or a proximal rim surface (81) and an inner circumference delimiting an interior spacing;
applying a pressure differential with a greater pressure on an interior side of the tubular sleeve (8) relative to an exterior side of the tubular sleeve (8), so as to increase an inner circumference of the tubular sleeve (8);
inserting the bending section (4) so into the interior spacing of the tubular sleeve (8) that the tubular sleeve (8) only covers or overlaps with at least a portion of the sleeve surface (90); and
relieving the pressure differential to decrease the inner circumference of the tubular sleeve (8), so that the tubular sleeve (8) covers the intermediate segments (42) and the rim surface (82) of the tubular sleeve (8) faces the flange surface (91) of the sub-assembly (50).

2. A method according to claim 1, wherein the bendable articulated tip part (5) is a bendable articulated tip part for an endoscope (1), the articulated tip part (5) comprising:
a sub-assembly (50) including a bending section (4) comprising a number of hingedly connected segments (41, 42, 43) including a proximal end segment (43), a distal end segment (41), and a plurality of intermediate segments (42) positioned between the proximal end segment (43) and the distal end segment (41), the sub-assembly (50) further including a circumferentially extending, outer flange surface (91, 92) which extends from a first outer circumference (91a) of the sub-assembly (50) to a second, smaller, outer circumference (91b) of the sub-assembly (50); and
a tubular sleeve (8) including a rim surface (81, 82), the tubular sleeve (8) at least covering the intermediate segments (42) of the bending section (4);
wherein the rim surface (81, 82) of the tubular sleeve faces the flange surface (91) of the sub-assembly (50).

3. A method according to claim 2, wherein the sub-assembly (50) includes a proximal, circumferentially extending, outer flange surface (91) which extends from a first outer circumference (91b) of the sub-assembly (50) to a second, smaller, outer circumference (91a) of the sub-assembly (50), the proximal flange surface (91) being positioned at or adjacent to the proximal end segment (43).

4. A method according to claim 3, wherein the proximal flange surface (91) is forming part of, potentially forming an integral part of or integrally formed in one piece by, an outer, circumferentially extending surface (91a) of the proximal end segment (43).

5. A method according to claim 3 or 4, wherein the tip part (5) further comprises a flexible tube (7) having a distal end (7b) connected to a proximal end (43a) of the proximal end segment (43), wherein a proximal rim surface (81) of the tubular sleeve (8) and/or the proximal flange surface (91) is/are positioned distally in relation to the distal end (7b) of the flexible tube (7).

6. A method according to any one of claims 2 to 5, wherein the sub-assembly (50) includes a distal, circumferentially extending, outer flange surface which extends from a third outer circumference (92a) of the sub-assembly (50) to a fourth, smaller, outer circumference (92b) of the sub-assembly (50), the distal flange surface (92) being positioned at or adjacent to the distal end segment (41).

7. A method according to claim 6, wherein the distal flange surface (92) forms part of, potentially forms, an integral part of or formed in one piece with, an outer, circumferentially extending surface of the distal end segment (41).

8. A method according to claim 6 or 7, the tip part further comprising a camera (61) assembly, wherein a distal rim surface (82) of the tubular sleeve (8) and/or the distal flange surface (92) is/are positioned proximally in relation to an image sensor (61a) of the camera assembly (61) and/or a light source (61b) of the camera assembly (61).

9. A method according to any one of claims 6 to 8, the tip part further comprising a cap (6) having a proximal end connected to the distal end (41a) of the distal end segment (41),
wherein a distal rim surface (82) of the tubular sleeve (8) and/or the distal flange surface (92) is/are positioned distally in relation to the proximal end (6b) of the cap (6).

10. A method according to any one of claims 6 to 9, the tip part further comprising a cap (6) having a proximal end (6b) connected to the distal end (41a) of the distal end segment (41), wherein the distal flange surface (92) is formed by an overlap of the distal end segment (41) and the cap (6) or formed by the interconnection of the distal end (41a) of the distal end segment (41) and the proximal end (6b) of the cap (6).

11. A method according to any one of the claims 2-10, the tip part (5) further comprising a cap (6) having a proximal end (6b) connected to the distal end (41a) of the distal end segment (41), wherein the tubular sleeve (8) does not overlap or cover a portion of the cap (6) and/or a portion of the flexible tube (7).

12. A method according to any one of claims 2 to 11, wherein the tubular sleeve (8) is secured to the bending section (4), potentially by an adhesive.

13. A method of manufacture of an endoscope (1) involving a method according to any one of the previous claims.

14. A method of manufacture of a system for visually inspecting inaccessible places such as human body cavities, the method comprising:
manufacturing an endoscope (1) according to the method of claim 13, and
connecting the endoscope (1) to a monitor (11),
wherein the monitor (11) allows an operator to view an image captured by the camera assembly (61) of the endoscope (1).

## Patentansprüche

1. Verfahren zum Zusammenbauen eines biegbaren gelenkigen Spitzenteils (5) für ein Endoskop (1), wobei das Verfahren die Schritte umfasst:
Bereitstellen einer Teilbaugruppe (50), die einen Biegeabschnitt (4) aufweist, der mehrere gelenkig verbundene Segmente (41, 42, 43) umfassend ein proximales Endsegment (43), ein distales Endsegment (41) und eine Vielzahl von Zwischensegmenten (42), die zwischen dem proximalen Endsegment (43) und dem distalen Endsegment (41) angeordnet sind, umfasst, wobei die Teilbaugruppe (50) ferner eine in Umfangsrichtung verlaufende äußere Flanschfläche (91) aufweist, die von einem ersten Außenumfang (91a) der Teilbaugruppe (50) zu einem zweiten, kleineren Außenumfang (91b) der Teilbaugruppe (50) verläuft, wobei die Teilbaugruppe (50) eine Hülsenoberfläche (90) aufweist, die eine in Umfangsrichtung verlaufende Außenfläche von jedem der Zwischensegmente (42) umfasst und an einem Ende durch den zweiten Umfang (91 b) begrenzt ist;
Bereitstellen einer rohrförmigen Hülse (8), die eine distale Randfläche (82) und/oder eine proximale Randfläche (81) und einen Innenumfang, der einen inneren Zwischenraum begrenzt, umfasst;
Anlegen einer Druckdifferenz mit einem größeren Druck an einer Innenseite der rohrförmigen Hülse (8) relativ zu einer Außenseite der rohrförmigen Hülse (8), um einen Innenumfang der rohrförmigen Hülse (8) zu vergrößern;
Einsetzen des Biegeabschnitts (4) in den inneren Zwischenraum der rohrförmigen Hülse (8), so dass die rohrförmige Hülse (8) nur wenigstens einen Teil der Hülsenoberfläche (90) bedeckt oder diesen überlappt; und
Lösen der Druckdifferenz, um den Innenumfang der rohrförmigen Hülse (8) zu verringern, so dass die rohrförmige Hülse (8) die Zwischensegmente (42) bedeckt und die Randfläche (82) der rohrförmigen Hülse (8) der Flanschfläche (91) der Teilbaugruppe (50) zugewandt ist.

2. Verfahren nach Anspruch 1, wobei das biegbare gelenkige Spitzenteil (5) ein biegbares gelenkiges Spitzenteil für ein Endoskop (1) ist, wobei das gelenkige Spitzenteil (5) umfasst:
eine Teilbaugruppe (50), die einen Biegeabschnitt (4) umfasst, der mehrere gelenkig verbundene Segmente (41, 42, 43) umfasst, die ein proximales Endsegment (43), ein distales Endsegment (41) und eine Vielzahl von Zwischensegmenten (42), die zwischen dem proximalen Endsegment (43) und dem distalen Endsegment (41) angeordnet sind, umfasst, wobei die Teilbaugruppe (50) ferner eine in Umfangsrichtung verlaufende äußere Flanschfläche (91, 92) aufweist, die von einem ersten Außenumfang (91a) der Teilbaugruppe (50) zu einem zweiten, kleineren Außenumfang (91b) der Teilbaugruppe (50) verläuft; und
eine rohrförmige Hülse (8) mit einer Randfläche (81, 82), wobei die rohrförmige Hülse (8) wenigstens die Zwischensegmente (42) des Biegeabschnitts (4) bedeckt; wobei die Randfläche (81, 82) der rohrförmigen Hülse der Flanschfläche (91) der Teilbaugruppe (50) zugewandt ist.

3. Verfahren nach Anspruch 2, wobei die Teilbaugruppe (50) eine proximale, in Umfangsrichtung verlaufende äußere Flanschfläche (91) aufweist, die von einem ersten Außenumfang (91b) der Teilbaugruppe (50) zu einem zweiten, kleineren Außenumfang (91a) der Teilbaugruppe (50) verläuft, wobei die proximale Flanschfläche (91) an dem proximalen Endsegment (43) oder an dieses angrenzend angeordnet ist.

4. Verfahren nach Anspruch 3, wobei die proximale Flanschfläche (91) einen Teil einer äußeren, in Umfangsrichtung verlaufenden Fläche (91a) des proximalen Endsegments (43) bildet, gegebenenfalls einen integralen Teil davon bildet oder integral einstückig damit gebildet ist.

5. Verfahren nach Anspruch 3 oder 4, wobei das Spitzenteil (5) ferner einen flexiblen Schlauch (7) mit einem distalen Ende (7b) umfasst, das mit einem proximalen Ende (43a) des proximalen Endsegments (43) verbunden ist, wobei eine proximale Randfläche (81) der rohrförmigen Hülse (8) und/oder die proximale Flanschfläche (91) distal bezogen auf das distale Ende (7b) des flexiblen Schlauchs (7) angeordnet ist/sind.

6. Verfahren nach einem der Ansprüche 2 bis 5, wobei die Teilbaugruppe (50) eine distale, in Umfangsrichtung verlaufende äußere Flanschfläche aufweist, die von einem dritten Außenumfang (92a) der Teilbaugruppe (50) zu einem vierten, kleineren Außenumfang (92b) der Teilbaugruppe (50) verläuft, wobei die distale Flanschfläche (92) an dem distalen Endsegment (41) oder an dieses angrenzend angeordnet ist.

7. Verfahren nach Anspruch 6, wobei die distale Flanschfläche (92) einen Teil einer äußeren, in Umfangsrichtung verlaufenden Fläche des distalen Endsegments (41) bildet, gegebenenfalls einen integralen Teil davon bildet oder einstückig damit gebildet ist.

8. Verfahren nach Anspruch 6 oder 7, wobei das Spitzenteil ferner eine Kamerabaugruppe (61) umfasst, wobei eine distale Randfläche (82) der rohrförmigen Hülse (8) und/oder die distale Flanschfläche (92) proximal bezogen auf einen Bildsensor (61a) der Kamerabaugruppe (61) und/oder eine Lichtquelle (61b) der Kamerabaugruppe (61) angeordnet ist/sind.

9. Verfahren nach einem der Ansprüche 6 bis 8, wobei das Spitzenteil ferner eine Kappe (6) mit einem proximalen Ende, das mit dem distalen Ende (41a) des distalen Endsegments (41) verbunden ist, umfasst,
wobei eine distale Randfläche (82) der rohrförmigen Hülse (8) und/oder die distale Flanschfläche (92) distal bezogen auf das proximale Ende (6b) der Kappe (6) angeordnet ist/sind.

10. Verfahren nach einem der Ansprüche 6 bis 9, wobei das Spitzenteil ferner eine Kappe (6) mit einem proximalen Ende (6b), das mit dem distalen Ende (41a) des distalen Endsegments (41) verbunden ist, umfasst, wobei die distale Flanschfläche (92) durch eine Überlappung des distalen Endsegments (41) und der Kappe (6) gebildet ist oder durch die Zwischenverbindung des distalen Endes (41a) des distalen Endsegments (41) und des proximalen Endes (6b) der Kappe (6) gebildet ist.

11. Verfahren nach einem der Ansprüche 2-10, wobei das Spitzenteil (5) ferner eine Kappe (6) mit einem proximalen Ende (6b), das mit dem distalen Ende (41a) des distalen Endsegments (41) verbunden ist, umfasst, wobei die rohrförmige Hülse (8) einen Abschnitt der Kappe (6) und/oder einen Abschnitt des flexiblen Schlauchs (7) nicht überlappt oder bedeckt.

12. Verfahren nach einem der Ansprüche 2 bis 11, wobei die rohrförmige Hülse (8) an dem Biegeabschnitt (4) befestigt wird, gegebenenfalls durch einen Klebstoff.

13. Verfahren zur Herstellung eines Endoskops (1), umfassend ein Verfahren nach einem der vorstehenden Ansprüche.

14. Verfahren zur Herstellung eines Systems zum visuellen Inspizieren von unzugänglichen Orten, wie z. B. Körperhöhlen des Menschen, wobei das Verfahren umfasst:
Herstellen eines Endoskops (1) gemäß dem Verfahren nach Anspruch 13 und
Verbinden des Endoskops (1) mit einem Monitor (11),
wobei der Monitor (11) einem Bediener ermöglicht, ein von der Kamerabaugruppe (61) des Endoskops (1) aufgenommenes Bild zu betrachten.

## Revendications

1. Procédé d'assemblage d'une partie de pointe articulée pliable (5) pour un endoscope (1), le procédé comprenant les étapes suivantes :
la fourniture d'un sous-ensemble (50) comprenant une section de pliage (4) comprenant un certain nombre de segments reliés par charnière (41, 42, 43) comprenant un segment d'extrémité proximale (43), un segment d'extrémité distale (41) et une pluralité de segments intermédiaires (42) positionnés entre le segment d'extrémité proximale (43) et le segment d'extrémité distale (41), le sous-ensemble (50) comprenant en outre une surface de bride extérieure (91) s'étendant circonférentiellement d'une première circonférence extérieure (91a) du sous-ensemble (50) jusqu'à une deuxième circonférence extérieure, plus petite, (91b) du sous-ensemble (50), où le sous-ensemble (50) comprend une surface de manchon (90) qui comprend une surface extérieure s'étendant circonférentiellement de chacun des segments intermédiaires (42) et est délimitée à une extrémité par la deuxième circonférence (91b) ;
la fourniture d'un manchon tubulaire (8) comprenant une surface de rebord distale (82) et/ou une surface de rebord proximale (81) et une circonférence intérieure délimitant un espacement intérieur ;
l'application d'une différence de pression avec une pression plus grande sur un côté intérieur du manchon tubulaire (8) par rapport à un côté extérieur du manchon tubulaire (8), de manière à augmenter une circonférence intérieure du manchon tubulaire (8) ;
l'insertion de la section de pliage (4) dans l'espacement intérieur du manchon tubulaire (8) de sorte que le manchon tubulaire (8) ne recouvre ou ne chevauche qu'au moins une partie de la surface de manchon (90) ; et
la réduction de la différence de pression pour diminuer la circonférence intérieure du manchon tubulaire (8), de sorte que le manchon tubulaire (8) recouvre les segments intermédiaires (42) et la surface de rebord (82) du manchon tubulaire (8) fait face à la surface de bride (91) du sous-ensemble (50).

2. Procédé selon la revendication 1, la partie de pointe articulée pliable (5) étant une partie de pointe articulée pliable pour un endoscope (1), la partie de pointe articulée (5) comprenant :
un sous-ensemble (50) comprenant une section de pliage (4) comprenant un certain nombre de segments reliés par charnière (41, 42, 43) comprenant un segment d'extrémité proximale (43), un segment d'extrémité distale (41), et une pluralité de segments intermédiaires (42) positionnés entre le segment d'extrémité proximale (43) et le segment d'extrémité distale (41), le sous-ensemble (50) comprenant en outre une surface de bride extérieure s'étendant circonférentiellement (91, 92) qui s'étend d'une première circonférence extérieure (91a) du sous-ensemble (50) jusqu'à une deuxième circonférence extérieure plus petite (91b) du sous-ensemble (50) ; et
un manchon tubulaire (8) comprenant une surface de rebord (81, 82), le manchon tubulaire (8) recouvrant au moins les segments intermédiaires (42) de la section de pliage (4) ;
la surface de rebord (81, 82) du manchon tubulaire faisant face à la surface de bride (91) du sous-ensemble (50).

3. Procédé selon la revendication 2, le sous-ensemble (50) comprenant une surface de bride extérieure proximale (91) s'étendant circonférentiellement qui s'étend d'une première circonférence extérieure (91b) du sous-ensemble (50) jusqu'à une deuxième circonférence extérieure plus petite (91a) du sous-ensemble (50), la surface de bride proximale (91) étant positionnée au niveau ou adjacente au segment d'extrémité proximale (43).

4. Procédé selon la revendication 3, la surface de bride proximale (91) formant partie d'une surface extérieure s'étendant circonférentiellement (91a) du segment d'extrémité proximale (43), formant potentiellement une partie intégrante de celle-ci ou étant formée d'une seule pièce avec celle-ci.

5. Procédé selon la revendication 3 ou 4, la partie de pointe (5) comprenant en outre un tube flexible (7) ayant une extrémité distale (7b) reliée à une extrémité proximale (43a) du segment d'extrémité proximale (43), une surface de rebord proximale (81) du manchon tubulaire (8) et/ou la surface de bride proximale (91) étant positionnée(s) distalement par rapport à l'extrémité distale (7b) du tube flexible (7).

6. Procédé selon l'une quelconque des revendications 2 à 5, le sous-ensemble (50) comprenant une surface de bride extérieure distale s'étendant circonférentiellement qui s'étend d'une troisième circonférence extérieure (92a) du sous-ensemble (50) à une quatrième circonférence extérieure plus petite (92b) du sous-ensemble (50), la surface de bride distale (92) étant positionnée au niveau ou adjacente au segment d'extrémité distale (41).

7. Procédé selon la revendication 6, la surface de bride distale (92) formant partie d'une surface extérieure s'étendant circonférentiellement du segment d'extrémité distale (41), formant potentiellement une partie intégrante de celle-ci ou étant formée d'une seule pièce avec celle-ci.

8. Procédé selon la revendication 6 ou 7, la partie de pointe comprenant en outre un ensemble de caméra (61), une surface de rebord distale (82) du manchon tubulaire (8) et/ou la surface de bride distale (92) étant positionnée(s) proximalement par rapport à un capteur d'image (61a) de l'ensemble de caméra (61) et/ou une source de lumière (61b) de l'ensemble de caméra (61).

9. Procédé selon l'une quelconque des revendications 6 à 8, la partie de pointe comprenant en outre un capuchon (6) ayant une extrémité proximale reliée à l'extrémité distale (41a) du segment d'extrémité distale (41),
une surface de rebord distale (82) du manchon tubulaire (8) et/ou la surface de bride distale (92) étant positionnée(s) distalement par rapport à l'extrémité proximale (6b) du capuchon (6).

10. Procédé selon l'une quelconque des revendications 6 à 9, la partie de pointe comprenant en outre un capuchon (6) ayant une extrémité proximale (6b) reliée à l'extrémité distale (41a) du segment d'extrémité distale (41), la surface de bride distale (92) étant formée par un chevauchement du segment d'extrémité distale (41) et du capuchon (6) ou formée par l'interconnexion de l'extrémité distale (41a) du segment d'extrémité distale (41) et de l'extrémité proximale (6b) du capuchon (6).

11. Procédé selon l'une quelconque des revendications 2 à 10, la partie de pointe (5) comprenant en outre un capuchon (6) ayant une extrémité proximale (6b) reliée à l'extrémité distale (41a) du segment d'extrémité distale (41), le manchon tubulaire (8) ne chevauchant pas ou ne recouvrant pas une partie du capuchon (6) et/ou une partie du tube flexible (7).

12. Procédé selon l'une quelconque des revendications 2 à 11, le manchon tubulaire (8) étant fixé à la section de pliage (4), potentiellement par un adhésif.

13. Procédé de fabrication d'un endoscope (1) impliquant un procédé selon l'une quelconque des revendications précédentes.

14. Procédé de fabrication d'un système permettant l'inspection visuelle d'endroits inaccessibles tels que des cavités corporelles humaines, le procédé comprenant :
la fabrication d'un endoscope (1) selon le procédé selon la revendication 13, et
la connexion de l'endoscope (1) à un moniteur (11),
le moniteur (11) permettant à un opérateur de visualiser une image capturée par l'ensemble de caméra (61) de l'endoscope (1).
